# EUROPEAN PATENT SPECIFICATION

(11) **EP 1 234 832 B1**
(45) Date of publication and mention of the grant of the patent: **13.02.2008**
(21) Application number: 02003403.9
(22) Date of filing: 04.12.1998
(51) Int. Cl.: C07H 1/08, C12N 15/10, C12Q 1/68

(54) **Isolation of nucleic acids**
Isolierung von Nukleinsäuren
Isolation d'acides nucléiques

(30) Priority: 06.12.1997 GB 9725839; 17.07.1998 GB 9815541
(43) Date of publication of application: 28.08.2002
(62) Divisional of application: 98957019.7
(73) Proprietor: INVITROGEN CORPORATION, Carlsbad, CA 92008 (US)
(72) Inventor: Baker, Matthew John, Maidstone, Kent ME15 9UJ (GB)
(74) Representative: Williams, Richard Andrew Norman

(56) References cited:
- EP-A- 0 301 899
- EP-A- 0 707 077
- EP-A- 0 832 897
- EP-A- 0 897 978
- WO-A-95/27718
- WO-A-96/09116
- WO-A-97/29825
- DATABASE WPI Section Ch, Week 9819 Derwent Publications Ltd., London, GB; Class B04, AN 98-210396 XP002106739 & JP 10 057056 A (ASAHI OPTICAL CO LTD), 3 March 1998 (1998-03-03)

## Description

The present invention relates to a method for extracting nucleic acids from biological material, particularly blood.

There is a very large demand for DNA analysis for a range of purposes and this has lead to the requirement for quick, safe, high throughput methods for the isolation and purification of DNA and other nucleic acids.

Samples for use for DNA identification or analysis can be taken from a wide range of sources such as biological material such as animal and plant cells, faeces, tissue etc. also samples can be taken from soil, foodstuffs, water etc.

Existing methods for the extraction of DNA include the use of phenol/chloroform, salting out, the use of chaotropic salts and silica resins, the use of affinity resins, ion exchange chromatography and the use of magnetic beads. Methods are described in US Patents 5057426, 4923978, EP Patents 0512767 Aland EP0515484B and WO 95/13368, WO 97/10331 and WO 96/18731. These patents and patent applications disclose methods of adsorbing nucleic acids on to a solid support and then isolating the nucleic acids. The previously used methods use some type of solvent to isolate the nucleic acids and these solvents are flammable, combustible or toxic.

EP 0 707077A describes the capture and selective release of nucleic acid from a lysate by using a water-soluble, weakly basic polymer to form a precipitate with the polymer The precipitate is then separated and the nucleic acid released from the polymer using extremely high salt conditions, strong base or heating.

Blood is one of the most abundant sample sources for DNA analysis as blood samples are routinely taken for a wide range of reasons. However because of the viscous and proteinaceous nature of blood using known DNA extraction methods it has proved difficult to accomplish using automation due to the difficulties of handling large volumes containing relatively small amounts of DNA. Hitherto nucleic acid extraction has been partially automated only by using hazardous reagents and slow processing times.

I have now devised an improved method for the extraction of nucleic acids and other biomolecules from blood and other biological materials.

Accordingly, the present invention provides a method of extracting nucleic acid from a cell lysate as set out in the claims.

The method is particularly useful if the biological material is blood, but the method can be used for a range of applications substances such as Plasmid and vector isolation and plant DNA extraction.

Preferably the cells in the blood are lysed to release nucleic acids and known lysing agents and methods can be used, such as contacting with ionic and non ionic detergents, hypotonic solutions of salts, proteases, chaotropic agents, solvents, using pH changes or heat. A method of lysing cells to isolate nucleic acid is described in WO 96/00228.

When the biological material consists of blood the samples can optionally be diluted with water or other diluent in order to make it easier to manipulate and to process.

Dilutions up to ten times can be used and in general more dilution can be better and it is a feature of the present invention that it allows low dilution of blood to be possible.

The solid phase with which the blood is contacted, can be a formed of a material which has a natural affinity for nucleic acids or it can be formed of a material which has its surface treated with an agent which will cause nucleic acids to bind to it or increase its affinity for nucleic acids. Suitable materials include controlled pore glass, polysaccharide (agarose or cellulose), other types of silica/glass, ceramic materials, porous plastic materials such as porous plastic plugs which in a single moulded part or as an insert in a standard tube, polystyrene beads para magnetic beads etc. The size and porosity is not critical and can vary and be selected for particular applications.

Suitable means for treating the surface of the solid phase or for derivitising it include treating it with a substance which can introduce a charge e.g. a positive charge on the surface or a hydrophilic or hydrophobic surface on the solid phase e.g. hydroxyl groups, nitrate groups, autoreactive groups, dyes and other aromatic compounds.

In a preferred embodiment of the invention the solid phase will cause DNA to be bound to it at one pH in preference to contaminants in the blood sample and will allow the bound nucleic acid to be released when it is contacted with an eluant at a different pH. This system can be used with a solid phase which incorporates histidine or a polyhistidine which will tend to bind nucleic acids at low pH e.g. less than 6 and will then release the bound nucleic acids when the pH is increased e.g. to greater than 8. Alternatively the nucleic acids are bound at substantially neutral pH to an aminated surface and released at very high pH.

In another embodiment of the invention a plastic moulding can incorporate a binding agent e.g. in a well in a plate etc. so that the binding agent is incorporated in the surface, the blood sample is then contacted with the surface so as to cause nucleic acids to be bound to the surface. The blood sample is then removed and the surface treated with an eluting agent to release the bound nucleic acids. When the surface is part of a well in a multi- well plate, the total system can be readily adapted for rapid large scale sampling and extraction techniques.

Binding agents which can be used include charge switchable ion exchange resins using a positively charged solid phase that can be reversed or made neutral by changing the pH above its pKa. e.g. nucleotides, polyamines, imidazole groups and other similar reagents with a suitable pKa value.

Also, nucleic acids can be bound by intercalation using a variety of intercalating compounds incorporated into the solid phase e.g. Actinomycin D, Ethidium Bromide etc.

In a further embodiment of the invention a plastic surface can be modified to include functional groups. The plastic can be any plastic used for containing samples e.g. polypropylene. The functional groups can be positively or negatively charged so as to bind the nucleic acids in the correct buffer solution.

Alternatively the functional groups can be chemical groups capable of covalent coupling to other ligands or polymers.

When the plastic is used in a plastic moulding e.g. in a well in a plate, or as a Polymerase Chain Reaction (PCR) tube, the surface characteristics of the plastic can be suitably modified for use in the present invention by including or adding the appropriate chemicals in the moulding compound e.g. as in an injection moulding compound.

When this is used in a PCR tube or in a deep well plate the tubes or wells can be used to isolate and immobilise small quantities of DNA or RNA generating a pure template for subsequent PCR or other genetic analysis and manipulation.

When the plastic is polypropylene e.g. it is in the form of a thin walled PCR tube the polypropylene surface can be modified by oxidising the surface with an oxidising agent such as potassium permanganate and sulphuric acid to create a carboxylated surface (COOH groups). This tube can then be used to improve the isolation of DNA from solutions or from crude samples e.g. blood. By adjusting the pH, di-electric constant, solubility or ionic strength the DNA or RNA can be immobilised on the walls of the tube, washed free of contaminants, ready for PCR or other analytical techniques.

The carboxy groups can be further modified by covalently coupling an anionic group such as imidazole or polyhistidine or any strong or weak ion exchanger, to allow binding of nucleic acids by a charge interaction. This tube could then be used to improve the isolation of DNA from solutions or from crude samples e.g. of blood. Again by adjusting the pH, di-electric constant, or ionic strength the DNA or RNA can be immobilised on the walls of the tube, washed free of contaminants, ready for PCR or other analytical techniques.

The nucleic acids can be eluted with in a low salt buffer so that it is ready for PCR or other analysis.

The solid phase can be contacted with a blood sample by mixing with the solid phase in a mixing/ stirring device, by passing the blood sample over the solid phase or the solid phase can be paramagnetic and manipulated by a magnetic field. Although the invention is particularly suitable for the separation or isolation of nucleic acids from blood it can be used with a range of biomolecules particularly those that require removal of cell wall debris or insoluble particles.

In a preferred embodiment of the invention the solid phase is in granular form in a column and the blood sample is drawn up through the column by means of a pressure differential being applied through the column, the blood sample is drawn up with air and the granular solid material can become fluidised thus increasing the mixing and contacting rates and minimising clogging.

The method of the invention is suitable for use in a multi-well format when a series of extractions from different samples can take place substantially simultaneously and this will facilitate the automation of the extraction process allowing rapid high throughput extraction to take place and to allow combinational chemistry to be performed. This will enable there to be a high throughput in a standard well array e.g. an eight by twelve array so that a large number of sample types can be treated automatically at the same time.

The invention is described in the Example.

### Example 1

Extraction of Nucleic Acids from Whole Blood

A charge switchable ion-exchanger was prepared by covalently coupling polyhistidine to 100 µm glass beads using glutaldehyde by mixing 1 gram of the aminated glass beads with 0.01 %(v/v) glutaldehyde in O.1M sodium bicarbonate at pH8 containing 20mg polyhistidine. After overnight incubation the beads were washed exhaustively to remove non-covalently bound material and stored in 10mM MES, pH5 containing 0.1 % (v/v) Tween 20.

About 300mg of the 100 µm derivitised glass beads were added to a 1ml plastic column enclosed at both ends.

A blood sample was incubated with an equal volume of 10mM MES pH5, containing 1% Tween 20, proteases (200 µg/ml) and 1 mM EDTA. After digestion is complete the blood was sucked up the column containing the glass beads and the DNA became immobilised allowing the contaminating proteins to pass through to waste.

The glass beads containing the immobilised DNA were washed with a buffer comprising 10mM MES pH5, containing 1% Tween 20, and 1mM EDTA and this was repeated until the wash solution was colourless.

After washing, the beads were dried with air and DNA eluted with a small quantity of 10mM Tris HCI, pH 8.5 and collected in a sterile tube ready for analysis. Thus the DNA were separated from the blood.

For different biomolecules, the buffer etc. can be suitably modified.

### Example 2

One gram of carboxylated paramagnetic beads were washed in 50mM Imidazole buffer pH6 and then mixed with 100mg of polyhistidine in 50ml of 50mM Imidazole buffer pH 6. A chemical coupling agent was added (EDC) at a final concentration of 5mg per ml and mixed overnight. The beads were washed in water, 0.5M sodium chloride, 1% Tween 20, 100mM Tris HCl pH 8 and stored in 10mM MES, 0.1% Tween 20 pH 5.

To extract DNA from blood, 1mg of beads were mixed with blood diluted in 10% Tween 20 with 25mM MES, 1mM EDTA pH 5. The beads were separated with a magnet and washed by resuspending in 1mM MES, 0.1% Tween 20. To elute the DNA the beads were resuspended in 10mM Tris HCl pH 8.5 and separated with a magnet leaving the DNA in solution.

## Claims

1. A method of extracting nucleic acid from a cell lysate, which method comprises:
bringing the cell lysate into contact with a solid phase at a first pH at which the solid phase has a positive charge so that the nucleic acid binds to the solid phase in preference to contaminants in the cell lysate;
releasing the nucleic acid at a second, higher pH above the pKa of the solid phase to reverse or neutralise the positive charge to release the bound nucleic acid from the solid phase;
wherein the solid phase is controlled pore glass, a polysaccharide, a ceramic material, a porous plastic material, a plastic material, polystyrene beads, paramagnetic beads, or a porous plastic plug which is a single moulded part or is an insert for a container; and
wherein the nucleic acid is released using a low salt buffer.

2. The method of claim 1, wherein the cell lysate is plant cell or animal cell lysate.

3. The method of claim 1, wherein the nucleic acid is a plasmid.

4. The method of claim 1, wherein the nucleic acid is DNA.

5. The method of claim 1, wherein the nucleic acid is RNA.

6. The method of claim 1, wherein the cell lysate is a blood cell lysate.

7. The method of any one of the preceding claims, wherein the plastic material is in the form of a pipette tip, a well plate or deep well plate, or a PCR tube.

8. The method of any one of the preceding claims, wherein the eluted nucleic acid is ready for analysis or amplification using PCR without the need for further purification.

9. The method of any one of preceding claims, wherein the nucleic acid binds to the solid phase at a pH of less than 6 and is released from the solid phase at a pH of greater than 8.

10. The method of any one of the preceding claims, wherein the nucleic acid binds to the solid phase at a substantially neutral pH and is released from the solid phase at a pH of greater than 10.

11. The method of any one of the preceding claims wherein the solid phase is formed of a material which has a natural affinity for nucleic acids

12. The method of any one of the preceding claims, wherein the solid phase is formed of a material which has its surface treated with an agent which introduces a positive charge to cause nucleic acids to bind to it or to increase its affinity for nucleic acids.

13. The method of any one of the preceding claims, wherein the solid phase is a charge switchable ion exchange resin having a positive charge that can be reversed or neutralised by changing the pH above its pKa.

14. The method of claim 13, wherein the ion exchange resin comprises a nucleotide, a polyamine or a compound containing an imidazole moiety.

15. The method of claim 12, wherein the surface of the solid material is treated with histidine or a polyhistidine.

16. The method of any one of the preceding claims, wherein the solid phase comprises a plastic surface modified to include functional groups.

17. The method of claim 15 or claim 16, wherein the plastic material is polypropylene.

18. The method of claim 17, wherein the polypropylene surface is modified by oxidising the surface with an oxidising agent to create a carboxylated surface.

19. The method of claim 18, wherein the carboxyl groups are further modified by covalently coupling an anion exchange group to allow binding of nucleic acids by a charge interaction.

20. The method of claim 19, wherein the anionic exchange group is imidazole, polyhistidine or a strong or weak ion exchanger.

21. The method of any one of the preceding claims, wherein the solid phase is a PCR tube or a well plate which is used to isolate and immobilise small quantities of nucleic acid, thereby generating a template for subsequent PCR or other genetic analysis and manipulation.

22. The method of any one of the preceding claim, the solid phase is a multi-well plate thereby allowing a series of extractions of nucleic acid from different samples to take place substantially simultaneously.

23. The method of any one of the proceeding claims, further comprising amplifying said released nucleic acid using PCR.

## Patentansprüche

1. Ein Verfahren zum Extrahieren von Nukleinsäure aus einem Zelllysat, wobei das Verfahren folgendes umfasst:
Herstellen eines Kontakts zwischen dem Zelllysat und einer Festphase bei einem ersten pH, bei dem die Festphase eine positive Ladung aufweist, so dass die Nukleinsäure eher an die Festphase als an Kontaminanten im Zelllysat bindet;
Ablösen der Nukleinsäure bei einem zweiten, höheren pH über dem pKₛ der Festphase, um die positive Ladung zu invertieren oder zu neutralisieren und so die gebundene Nukleinsäure von der Festphase abzulösen;
wobei es sich bei der Festphase um Controlled Pore Glass, ein Polysaccharid, ein keramisches Material, ein poröses Kunststoffmaterial, ein Kunststoffmaterial, Polystyrolkügelchen, paramagnetische Kügelchen oder einen porigen Kunststoffzapfen, der ein einzelnes Formteil oder einen Einsatz für einen Behälter darstellt, handelt; und
wobei die Nukleinsäure unter Verwendung eines Niedrigsalzpuffers gelöst wird.

2. Das Verfahren gemäß Anspruch 1 oder 2, wobei es sich bei dem Zelllysat um Pflanzenzelllysat oder Tierzelllysat handelt.

3. Das Verfahren gemäß Anspruch 1 oder 2, wobei es sich bei der Nukleinsäure um ein Plasmid handelt.

4. Das Verfahren gemäß Anspruch 1 oder 2, wobei es sich bei der Nukleinsäure um DNA handelt.

5. Das Verfahren gemäß Anspruch 1 oder 2, wobei es sich bei der Nukleinsäure um RNA handelt.

6. Das Verfahren gemäß Anspruch 1 oder 2, wobei es sich bei dem Zelllysat um ein Blutzelllysat handelt.

7. Das Verfahren gemäß Anspruch 1, wobei das Kunststoffmaterial in Form einer Pipettenspitze, einer Mikrotiterplatte oder einer Mikrotiterplatte mit tiefen Kavitäten oder eines PCR-Röhrchens vorliegt.

8. Das Verfahren gemäß Anspruch 1 oder Anspruch 6, wobei die eluierte Nukleinsäure für die Analyse oder Amplifikation unter Verwendung von PCR bereit steht, ohne dass weitere Reinigung erforderlich wäre.

9. Das Verfahren gemäß einem der vorhergehenden Ansprüche, wobei die Nukleinsäure bei einem pH von weniger als 6 an die Festphase bindet und bei einem pH von mehr als 8 von der Festphase abgelöst wird.

10. Das Verfahren gemäß einem der vorhergehenden Ansprüche, wobei die Nukleinsäure bei einem im Wesentlichen neutralen pH an die Festphase bindet und bei einem pH von mehr als 10 von der Festphase abgelöst wird.

11. Das Verfahren gemäß einem der vorhergehenden Ansprüche, wobei die Festphase aus einem Material gebildet wird, das eine natürliche Affinität für Nukleinsäuren aufweist.

12. Das Verfahren gemäß einem der vorhergehenden Ansprüche, wobei die Festphase aus einem Material gebildet wird, dessen Oberfläche mit einem Wirkstoff behandelt ist, der eine positive Ladung einführt, um zu bewirken, dass die Nukleinsäuren an dieses binden oder um die Affinität desselben für Nukleinsäuren zu erhöhen.

13. Das Verfahren gemäß einem der vorhergehenden Ansprüche, wobei es sich bei der Festphase um ein lonenaustauscherharz mit veränderbarer Ladung handelt, das eine positive Ladung aufweist, die durch Ändern des pHs auf einen Wert über dessen pKₛ invertiert oder neutralisiert werden kann.

14. Das Verfahren gemäß Anspruch 13, wobei das lonenaustauscherharz ein Nukleotid, ein Polyamin oder eine Verbindung, die eine Imidazolgruppe enthält, umfasst.

15. Das Verfahren gemäß Anspruch 12, wobei die Oberfläche der Festphase mit Histidin oder einem Polyhistidin behandelt ist.

16. Das Verfahren gemäß einem der vorhergehenden Ansprüche, wobei die Festphase eine Kunstoffoberfläche umfasst, die so modifiziert wurde, dass sie funktionelle Gruppen einschließt.

17. Das Verfahren gemäß Anspruch 15 oder Anspruch 16, wobei es sich bei dem Kunststoffmaterial um Polypropylen handelt.

18. Das Verfahren gemäß Anspruch 17, wobei die Poylpropylenoberfläche durch Oxidieren der Oberfläche mit einem Oxidationsmittel modifiziert ist, um eine carboxylierte Oberfläche zu schaffen.

19. Das Verfahren gemäß Anspruch 18, wobei die Carboxylgruppen weiter durch kovalente Kopplung einer Anionenaustauschergruppe modifiziert werden, um die Bindung von Nukleinsäuren durch eine Ladungswechselwirkung zu ermöglichen.

20. Das Verfahren gemäß Anspruch 19, wobei es sich bei der Anionenaustauschergruppe um Imidazol, Polyhistidin oder einen starken oder schwachen lonenaustauscher handelt.

21. Das Verfahren gemäß einem der vorhergehenden Ansprüche, wobei es sich bei der Festphase um ein PCR-Röhrchen oder eine Mikrotiterplatte handelt, die verwendet wird, um kleine Mengen an Nukleinsäure zu isolieren und zu immobilisieren, wodurch ein Templat für anschließende PCR oder sonstige genetische Analyse und Manipulation gebildet wird.

22. Das Verfahren gemäß einem der vohergehenden Ansprüche, wobei es sich bei der Festphase um eine Multiwellplatte handelt, wodurch ermöglicht wird, dass eine Reihe an Extraktionen von Nukleinsäure aus verschiedenen Proben im Wesentlichen gleichzeitig durchgeführt werden.

23. Das Verfahren gemäß einem der vorhergehenden Ansprüche, das des Weiteren das Amplifizieren dieser abgelösten Nukleinsäure unter Verwendung von PCR umfasst.

## Revendications

1. - Procédé d'extraction d'acide nucléique à partir d'un lysat cellulaire, lequel procédé comprend les étapes consistant à :
- amener le lysat cellulaire en contact avec une phase solide à un premier pH auquel la phase solide a une charge positive de telle sorte que l'acide nucléique se lie à la phase solide de préférence aux contaminants dans le lysat cellulaire ;
- libérer l'acide nucléique à un second pH, supérieur, au-dessus du pKa de la phase solide, pour inverser ou neutraliser la charge positive afin de libérer de la phase solide l'acide nucléique lié ;
procédé dans lequel la phase solide est un verre à pores contrôlés, un polysaccharide, une matière céramique, une matière plastique poreuse, une matière plastique, des perles de polystyrène, des perles paramagnétiques ou un bouchon en matière plastique poreuse, qui est une simple pièce moulée ou est un insert pour un conteneur ; et
dans lequel l'acide nucléique est libéré à l'aide d'un tampon à faible teneur en sel.

2. - Procédé selon l'une des revendications 1 ou 2, dans lequel le lysat cellulaire est un lysat de cellules végétales ou de cellules animales.

3. - Procédé selon l'une des revendications 1 ou 2, dans lequel l'acide nucléique est un plasmide.

4. - Procédé selon l'une des revendications 1 ou 2, dans lequel l'acide nucléique est l'ADN.

5. - Procédé selon l'une des revendications 1 ou 2, dans lequel l'acide nucléique est l'ARN.

6. - Procédé selon l'une des revendications 1 ou 2, dans lequel le lysat cellulaire est un lysat de cellules du sang.

7. - Procédé selon la revendication 1, dans lequel la matière plastique est sous la forme d'une pointe de pipette, d'une plaque à puits ou d'une plaque à puits profonds ou d'un tube de PCR.

8. - Procédé selon la revendication 1 ou la revendication 6, dans lequel l'acide nucléique élué est prêt pour une analyse ou une amplification à l'aide d'une PCR sans la nécessité d'une purification supplémentaire.

9. - Procédé selon l'une quelconque des revendications précédentes, dans lequel l'acide nucléique se lie à la phase solide à un pH de moins de 6 et est libéré de la phase solide à un pH de plus de 8.

10. - Procédé selon l'une quelconque des revendications précédentes, dans lequel l'acide nucléique se lie à la phase solide à un pH sensiblement neutre et est libéré de la phase solide à un pH de plus de 10.

11. - Procédé selon l'une quelconque des revendications précédentes, dans lequel la phase solide est formée d'une matière qui a une affinité naturelle pour les acides nucléiques.

12. - Procédé selon l'une quelconque des revendications précédentes, dans lequel la phase solide est formée d'une matière qui a sa surface traitée par un agent qui introduit une charge positive pour amener les acides nucléiques à s'y lier ou pour augmenter son affinité pour les acides nucléiques.

13. - Procédé selon l'une quelconque des revendications précédentes, dans lequel la phase solide est une résine échangeuse d'ions à charge commutable ayant une charge positive qui peut être inversée ou neutralisée par le changement du pH au-dessus de son pKa.

14. - Procédé selon la revendication 13, dans lequel la résine échangeuse d'ions comprend un nucléotide, une polyamine ou un composé contenant une fraction imidazole.

15. - Procédé selon la revendication 12, dans lequel la surface de la matière solide est traitée par de l'histidine ou une polyhistidine.

16. - Procédé selon l'une quelconque des revendications précédentes, dans lequel la phase solide comprend une surface de matière plastique modifiée pour comprendre des groupes fonctionnels.

17. - Procédé selon la revendication 15 ou la revendication 16, dans lequel la matière plastique est le polypropylène.

18. - Procédé selon la revendication 17, dans lequel la surface de polypropylène est modifiée par l'oxydation de la surface par un agent oxydant pour créer une surface carboxylée.

19. - Procédé selon la revendication 18, dans lequel les groupes carboxyle sont encore modifiés par le couplage covalent d'une groupe échangeur d'anions pour permettre une liaison d'acides nucléiques par une interaction de charges.

20. - Procédé selon la revendication 19, dans lequel le groupe échangeur d'anions est l'imidazole, la polyhistidine ou un échangeur d'ions fort ou faible.

21. - Procédé selon l'une quelconque des revendications précédentes, dans lequel la phase solide est un tube de PCR ou une plaque à puits qui est utilisé pour isoler et immobiliser de petites quantités d'acide nucléique, générant de cette façon une matrice pour une PCR ultérieure ou autre analyse et manipulation génétique.

22. - Procédé selon l'une quelconque des revendications précédentes, dans lequel la phase solide est une plaque à plusieurs puits, permettant de cette façon à une série d'extractions d'acide nucléique à partir de différents échantillons d'avoir lieu sensiblement simultanément.

23. - Procédé selon l'une quelconque des revendications précédentes, comprenant en outre l'amplification dudit acide nucléique libéré à l'aide d'une PCR.
